(19) Europäisches Patentamt
European Patent Office
Office européen des brevets

(11) **EP 2 358 774 B1**

(12) **FASCICULE DE BREVET EUROPEEN**

(45) Date de publication et mention de la délivrance du brevet:
**10.06.2015 Bulletin 2015/24**

(21) Numéro de dépôt: **09795470.5**

(22) Date de dépôt: **19.11.2009**

(51) Int Cl.:
*C08F 283/06* (2006.01)     *A61L 15/32* (2006.01)
*A61L 27/52* (2006.01)       *A61L 24/00* (2006.01)
*A61L 26/00* (2006.01)

(86) Numéro de dépôt international:
**PCT/FR2009/052224**

(87) Numéro de publication internationale:
**WO 2010/058132 (27.05.2010 Gazette 2010/21)**

(54) **MATERIAUX SOUS FORME DE RESEAUX INTERPENETRES DE POLYMERES ASSOCIANT UN GEL DE FIBRINE ET UN RESEAU DE POLYETHYLENE GLYCOL**

MATERIALIEN IN FORM VON INTERPENETRIERENDEN POLYMEREN NETZWERKEN MIT EINER KOMBINATION VON FIBRINGEL UND GLYKOL-POLYETHYLEN-NETZWERK

MATERIALS IN THE FORM OF INTERPENETRATING POLYMER NETWORKS COMBINING A FIBRIN GEL AND A GLYCOL POLYETHYLENE NETWORK

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **20.11.2008   FR 0806514**

(43) Date de publication de la demande:
**24.08.2011   Bulletin 2011/34**

(73) Titulaire: **Université de Cergy-Pontoise**
**95011 Cergy-Pontoise Cedex (FR)**

(72) Inventeurs:
• **LARRETA-GARDE, Véronique**
  **F-95290 L'Isle-Adam (FR)**
• **FICHET, Odile**
  **F-78300 Poissy (FR)**
• **AKPALO, Amivi, Edéfia**
  **F-94410 Saint-Maurice (FR)**

(74) Mandataire: **Novagraaf Technologies**
**Bâtiment O2**
**2, rue Sarah Bernhardt**
**CS90017**
**92665 Asnières-sur-Seine Cedex (FR)**

(56) Documents cités:
**WO-A-2004/014969     WO-A-2007/028258**

• **SCHMEDLEN, R.H. ET AL: "Photocrosslinkable polyvinyl alcohol hydrogels that can be modified with cell adhesion peptides for use in tissue engineering" BIOMATERIALS, vol. 23, 2002, pages 4325-4332, XP002532610 cité dans la demande**

Remarques:
Le dossier contient des informations techniques présentées postérieurement au dépôt de la demande et ne figurant pas dans le présent fascicule.

EP 2 358 774 B1

**Description**

**Domaine technique**

La présente invention se rapporte à un procédé de préparation d'un matériau sous forme de réseau interpénétré de polymères (RIP) associant un gel physique de fibrine et un réseau polyéthylène glycol (PEG).

Elle se rapporte également aux matériaux susceptibles d'être obtenus par ce procédé ainsi que leur utilisation comme pansement pour les plaies, pansement chirurgical, comme un dispositif pour délivrer des agents thérapeutiques, comme revêtement pour des dispositifs médicaux comme par exemple des stents, valves pour le coeur, des cathéters, des filtres prosthétiques vasculaires etc. Lesdits matériaux peuvent également être utilisés pour des cultures de cellules eucaryotes, ou encore comme support des molécules actives comme les agents de cicatrisation, facteurs de croissance, antibiotiques, bactéricides, bactériostatiques ou les enzymes.

Dans la description ci-dessous, les références entre crochets [] renvoient à la liste des références présentées à la fin du texte.

**Etat de l'art**

L'obtention d'un matériau se présentant sous forme d'un gel reste un réel besoin notamment dans le domaine médical. Les gels possèdent la propriété d'être des solides élastiques très riches en solvant. En particulier, pour les biogels ou gels obtenus à partir de molécules d'origine biologique, le solvant est l'eau. Leur volume et leur forme peuvent varier de manière réversible en fonction des stimuli environnementaux comme le pH, la température, la composition du solvant, la force ionique, les champs électriques et magnétiques. Ces biogels sont des réseaux hydrophiles qui retiennent de grandes quantités d'eau sans se dissoudre et ont ainsi des caractéristiques physiques similaires à celles des tissus biologiques mous [1].

L'importance des hydrogels (polymères réticulés ou biogels) dans le champ des biomatériaux est justifiée par la nature molle et élastomère des hydrogels qui minimise l'irritation mécanique et les frictions avec les tissus donc les processus inflammatoires. Les hydrogels ont une grande perméabilité à l'oxygène, aux nutriments et aux autres métabolites hydrosolubles favorisant la croissance de structures tissulaires plus épaisses [2]. Les hydrogels sont attrayants pour la délivrance de médicaments en raison de leur bonne compatibilité avec les composés hydrophiles et macromoléculaires (protéines, polysaccharides, oligonucléotides), de leur biocompatibilité et de la facilité à réguler la diffusion des médicaments en contrôlant leur gonflement, leur densité de réticulation et leur dégradation. Ces matériaux sont intéressants pour une diffusion localisée puisqu'ils peuvent être formés in situ et ainsi adhérer et être conformes aux tissus cibles. Cette utilisation pour une délivrance locale de principes actifs semble agir en synergie avec l'effet barrière des hydrogels [3]. Les hydrogels ont été étudiés comme matériaux potentiels pour la régénération de l'os, du tendon, des nerfs et essentiellement du cartilage.

Cependant, les gels formés à partir de molécules biologiques sont des matériaux mous, aussi leurs principaux inconvénients sont de présenter une faible tenue mécanique, d'être très déformables et difficile à manipuler.

Il subsiste donc un réel besoin à pouvoir préparer un matériau qui soit manipulable, qui ait les propriétés d'un gel de dimensions stables et une résistance améliorée à la dégradation par exemple par action de la chaleur ou des enzymes etc.

Dans le cadre de la présente invention, un « réseau » est une association de polymères. Cette association entre les polymères peut être assurée par des interactions fortes ou faibles (liaisons covalentes, liaisons hydrogène, Van der Waals etc). Dans le cas de liaisons covalentes, l'association est une réticulation et le polymère est dit « réticulé ».

La définition d'un gel inclut trois critères [4] :

- Les gels sont composés d'au moins deux composants : l'un très fortement majoritaire est le solvant et l'autre, minoritaire, est appelé solide.
- Les deux composants sont continus à travers tout le milieu. La phase dite solide, constitue un réseau qui emprisonne la phase dite liquide correspondant au solvant, et l'empêche de s'écouler.
- L'ensemble du milieu se comporte comme un solide mou facile à déformer.

Les gels sont classifiables selon le type de liens qui forment le réseau. Ainsi deux grands mécanismes de gélification se distinguent qui conduisent à des gels "physiques" ou à des gels "chimiques".

Un gel physique est un assemblage supramoléculaire constitué par des molécules liées entre elles par des liaisons de faible énergie (Van der Waals, liaisons hydrogènes, polaires, etc.). La stabilité de cet assemblage est associée à une plage précise de conditions physico-chimiques (pH, concentration en molécules, température, qualité du solvant, force ionique, etc.). En dehors de cette plage, le mélange est liquide. La transition sol/gel est donc réversible pour les gels physiques. Ainsi, une modification des paramètres du milieu peut entraîner la destruction de l'édifice et induire une transition gel-sol. Il est bien connu de l'art antérieur des compositions pour obtenir des gels "physiques". Les biogels sont obtenus essentiellement à partir de macromolécules ou polymères d'origine naturelle: protéines ou polysaccharides. On peut citer, à titre d'exemples de protéines susceptibles de gélifier, la gélatine, la fibrine, le collagène, l'actine, la myoglobine, les protéines du petit lait, notamment les caséines et la lactoglobuline, les protéines du soja, du blé et notamment la gliadine et la gluténine, du blanc d'oeuf et notamment l'ovalbumine. On peut citer, à titre d'exemples de polysaccharides susceptibles de gélifier les carraghénanes, les alginates, les pectines, le chitosan, la cellulose, la chitine, le glycogène ou encore l'amidon.

On connaît également dans l'état de l'art des gels qualifiés de "chimiques". Un gel chimique correspond à un assemblage supramoléculaire dont les molécules sont associées par des liaisons de forte énergie (covalentes). La stabilité de cet assemblage est donc très grande. Ces gels chimiques présentent une stabilité améliorée, le seul moyen d'effectuer une transition gel/solution consistant à détruire les liaisons covalentes du réseau. C'est pourquoi, la transition sol/gel des gels chimiques est dite irréversible.

Une famille de gels chimiques correspond aux gels catalysés enzymatiquement. Ce mode de gélification est surtout observé dans les grands processus biologiques. La coagulation sanguine, la cicatrisation, la formation de peau, l'assemblage des matrices extracellulaires sont des processus biologiques où le passage des protéines solubles à l'état de gel est indispensable. *In vivo,* un nombre limité d'enzymes par exemple les lysyloxydases, les transglutaminases, catalysent ces réactions. *In vitro,* la plus utilisée est la transglutaminase qui crée des ponts covalents entre les chaînes latérales des résidus lysine et glutamine des protéines [5].

Les propriétés structurales et biochimiques de la fibrine en font un candidat prometteur en ingénierie tissulaire et en médecine régénérative. La modification et la fonctionnalisation de gel de fibrine ont été utilisées pour le relargage contrôlé de gènes et de facteurs de croissance. De plus, la fibrine possède naturellement des sites de liaison aux cellules et a donc également été étudiée pour l'adhérence, l'étalement, la migration et la prolifération cellulaire. A cause des effets prouvés sur plusieurs types cellulaires, la fibrine est d'un intérêt particulier comme support en ingénierie tissulaire vasculaire [6].

La fibrine est obtenue à partir du fibrinogène, glycoprotéine de 340 kDa d'origine hépatique. Le fibrinogène est un polyélectrolyte anionique dans les conditions physiologiques et ne peut donc pas s'associer ou s'agréger. L'hydrolyse du fibrinogène par la thrombine, protéase d'origine sérique, conduit à la libération de petits fragments peptidiques et à l'obtention de fibrine. Les interactions intermoléculaires deviennent alors possibles, les molécules s'autoassocient, via des liaisons de faible énergie, créant ainsi un réseau de gel physique de fibrine.

Dans le processus de coagulation ou de cicatrisation, la stabilisation du gel physique est obtenue grâce à une transglutaminase sérique (facteur 13) créant des liaisons isopeptidiques covalentes entre les chaînes de fibrine conduisant à la formation d'un réseau chimique de fibrine insoluble.

Cependant, les gels chimiques de fibrine sont des matériaux mous, aussi leurs principaux inconvénients sont de présenter une faible tenue mécanique, d'être très déformables et difficile à manipuler.

Comme indiqué précédemment, il existe un besoin de disposer d'un matériau sous forme de gel dit « physique », qui soit stable et qui maintienne son intégrité structurale et donc ses propriétés durant toute la durée de l'utilisation qui en est faite, mais dont les éléments d'origine biologique soient réversibles (ou labiles) c'est-à-dire qui puissent être déstructurés et dégradés lorsque cela est souhaité. A ce jour, la plupart des procédés connus permettent la préparation de matériau sous forme de gel dit « chimique ». Une possibilité pour obtenir un gel auto-supporté présentant ces propriétés est d'introduire ce gel dans une architecture de réseau interpénétré ou semi-interpénétré de polymères (RIP ou semi-RIP).

Il existe donc un réel besoin d'un procédé palliant les insuffisances, défaut, limitations, inconvénients et obstacles de l'état de l'art, en particulier d'un procédé mettant en oeuvre un matériau sous forme de réseau interpénétré de polymères (RIP) associant un gel physique de fibrine et un réseau de polyéthylène glycol.

Ce procédé permet la préparation dudit réseau interpénétré de polymères (RIP) en une étape (« one pot » en anglais) dans des conditions opératoires respectant la nature de chaque polymère évitant ainsi leur dégradation.

Par ailleurs, ce procédé permet de maîtriser la formation dudit réseau *in situ,* pouvant ainsi fournir un matériau « sur mesure », adapté à l'application à laquelle il est destiné.

## Description de l'invention

La présente invention a précisément pour but de répondre à ce besoin en fournissant un procédé de préparation d'un matériau sous forme de réseau interpénétré de polymères (RIP) associant un gel physique de fibrine et un réseau de polyéthylène glycol (PEG), comprenant les étapes suivantes :

i) préparer un mélange en introduisant dans du tampon

- une solution de fibrinogène,

- des monomères de formule (I) :

$$X_1\text{-}(CH_2\text{-}CH_2\text{-}O)_n\text{-}X_2$$

dans laquelle $X_1$ et $X_2$, sont des groupements chimiques identiques ou différents, choisis dans le groupe comprenant le vinyle, l'acrylate, le méthacrylate ou l'allyle et n est un nombre compris entre 1 et 140, par exemple de 1 à 50, de préférence de 2 à 20,

- éventuellement des monomères de formule (II) choisis parmi les dérivés de polyéthylène glycol portant un groupement choisi dans le groupe comprenant -$CH_3$, -$NH_2$, -OH, -$CH_2CH_3$, le vinyle, l'acrylate, le méthacrylate ou l'allyle ; les dérivés d'acrylate, de méthacrylate et de styrène choisis dans le groupe comprenant l'hydroxyéthylméthacrylate, l'hydroxyéthylacrylate, l'acétate de vinyle, la N-vinyl pyrrolidone, la N-vinyl pyridine, l'acrylonitrile, l'acide acrylique, le styrène sulfonate de sodium, le bromure de (vinylbenzyl)triméthyl-ammonium, le chlorure de (vinylbenzyl)triméthyl-ammonium, le chlorure de triméthyl-vinyloxycarbonylméthyl-ammonium, le bromure de triméthyl-vinyloxycarbonylméthyl-ammonium, le bromure de triéthyl-2-vinyloxycarbonyl-éthyl)-ammonium, le bromure d'allyloxycarbonylméthyl-triméthyl ammonium, le poly(éthylène glycol) méthacrylate, le poly(éthylène glycol) acrylate,
- un amorceur de polymérisation ;

ii) préparer un mélange réactionnel en ajoutant une solution de thrombine au mélange préparé en i) ;

iii) incuber le mélange réactionnel obtenu en ii) à une température comprise entre 20 et 40 °C ;

iv) réaliser une polymérisation et réticulation des monomères de formule (I) avec éventuellement les monomères de formule (II).

Le procédé de l'invention permet de préparer un matériau sous forme de réseau interpénétré de polymères (RIP) qui est homogène, manipulable, stable dans le temps et de dimensions stables.

Le procédé selon l'invention, notamment à travers la température de l'incubation et la polymérisation, permet de contrôler la gélification et la polymérisation/réticulation des monomères. La gélification et la réticulation peuvent avoir lieu simultanément ou séquentiellement.

Par ailleurs, lorsque la polymérisation est une photopolymérisation, le matériau obtenu en sortie de synthèse (à l'issue de l'étape (iv)) est avantageusement un matériau stérile.

Dans le cadre de la présente invention, les termes polyéthylène glycol (PEG), polyoxyde d'éthylène ou polyoxyéthylène (POE) sont indifféremment utilisés pour désigner le réseau polyéthylène glycol obtenu par réticulation d'un mélange de monomères de formule (I) ou par polymérisation / réticulation d'un mélange de monomères de formules (I) et (II).

Les monomères de formules (I) peuvent être qualifiés de difonctionnel (portant deux fonctions réactives selon le mécanisme de réticulation proposé). Les monomères de formule (II) sont monofonctionnels (une seule fonction réactive selon le mécanisme de polymérisation proposé).

Dans le cadre de la présente invention, par "tampon" on entend une solution aqueuse dont le contenu en eau est d'au moins 30% en poids pouvant aller jusqu'au 99 % en poids total du solvant et qui maintient approximativement le même pH malgré l'addition de petites quantités d'un acide, d'une base ou d'une dilution. Le contenu en eau du tampon est en particulier de 70 à 98% en poids total du solvant.

Le tampon peut, en outre, comprendre d'autres solvants choisis dans le groupe comprenant du méthanol, de l'éthanol, de la pyridine, l'acétone, l'acide acétique, du DMSO, du benzène, du dichlorométhane.

A titre d'exemple de tampon, on peut citer les tampons phosphate, Hepes, Tris, sodium barbital, Tris-maléate.

Durant toute la durée du procédé, le pH du milieu aqueux est maintenu entre 6 et 8 au moyen d'une solution de tampon, par exemple une solution de trihydroxyaminométhane (Tris).

Le fibrinogène peut être par exemple du fibrinogène humain, de porc ou de boeuf.

Dans le procédé de l'invention les monomères de formules (I) peuvent être utilisés seuls ou en mélange avec des monomères (II) tels que définis précédemment.

Dans le cas des monomères de formule (I), n peut être défini comme le nombre moyen d'unités d'oxyéthylène (-$CH_2CH_2O$-) contenu dans lesdits monomères.

De préférence, les monomères de formule (I) sont ceux dans lesquels $X_1$ et $X_2$, sont des fonctions chimiques identiques ou différentes, choisis dans le groupe comprenant des méthacrylates ou acrylates et n est un nombre compris entre 2 et 20.

Les monomères de formule (I) peuvent, par exemple, être utilisés seuls. Ces monomères sont des précurseurs du réseau de polyéthylène glycol (PEG).

Le réseau (PEG) peut contenir entre 1 et 100% en masse de monomères de formule (I) et entre 99 et 0% en masse de monomères de formule (II). Le réseau (PEG) peut par exemple contenir entre 10 et 100% en masse de monomères de formule (I) et entre 90 et 0% en masse de monomères de formule (II).

Dans un mode de réalisation particulier de l'invention, lorsque l'on souhaite avoir un matériau présentant des propriétés bactéricides ou bactériostatiques, le monomère de formule (II) peut porter avantageusement un groupement ammonium.

Tout amorceur, générateur de radicaux libres connu de l'homme du métier, permettant la polymérisation peut convenir.

Par exemple, l'amorceur peut être un amorceur thermique choisi dans le groupe comprenant les peroxydes, les composés diazoïques ou les persulfates, par exemple le persulfate de potassium, le persulfate d'ammonium et le peroxyde d'hydrogène.

Par exemple, l'amorceur peut être un amorceur de photopolymérisation. Il peut être choisi dans le groupe comprenant les Irgacures (par exemple 2959, 184, 651, 819) [6], le 2-hydroxy-4-(2-hydroxyéthoxy)-2-méthylpropiophénone, la benzophénone, le 2, 4-diméthylbenzophénone, la benzoïne, les benzophénones ioniques choisis dans le groupe comprenant le chlorure de 4-triméthylméthylammonium benzophénone, le sel de sodium du 4-sulfométhylbenzyl, les cétones aromatiques et les aldéhydes notamment le benzaldéhyde, l'acétophénone, le biacétyle, le parachlorobenzophénone, l'acide ferrulique, et tous les composés produisant sous rayonnements UV/visible des radicaux. L'irgacure 2959 est choisi de préférence.

Les concentrations des différents composés dans le mélange (étape i) peuvent être par exemple :

- pour le fibrinogène de 1 à 50 mg/mL, de préférence entre 5 et 25 mg/mL,
- pour les monomères de formule (I) et éventuellement (II) de 1 à 400 mg/mL, de préférence 100 mg/mL de 0,1 à 1 mg/mL, de préférence 0,42 mg/mL,

La thrombine utilisée dans l'étape (ii) peut être issue par exemple du plasma humain, de porc ou de boeuf.

La concentration de la thrombine dans le mélange de étape (ii) peut être par exemple de 0,1 à 2,5 U/mL, de préférence de 0,2 à 1 U/mL.

Dans l'étape iii), la température d'incubation peut être par exemple comprise entre 30 et 40°C, de préférence de 37°C.

C'est au cours de cette étape que le gel physique de fibrine est formé à partir de fibrinogène par hydrolyse enzymatique. Cette hydrolyse enzymatique est catalysée par la thrombine. De manière inattendue, la formation du gel, l'activité de l'enzyme et les propriétés du gel ne sont pas altérées par la présence des monomères de formule (I) et éventuellement des monomères de formule (II), et leur polymérisation.

Lorsque l'amorceur est un amorceur thermique, la polymérisation des monomères de formules (I) et/ou (II) peut avoir lieu en même temps que l'étape (iii) ou après l'étape (iii). Lorsque la polymérisation a lieu après l'étape (iv), elle peut être réalisée par exemple à une température comprise entre 25 et 60°C. De préférence, la température est choisie de manière à ce que le gel de fibrine ne soit pas dénaturé.

La polymérisation thermique peut être réalisée entre 0,5 et 10 heures, de préférence entre 1 et 3 heures

Dans l'étape iv), lorsque l'amorceur est un amorceur de photopolymérisation, par exemple ceux précités, la polymérisation peut être réalisée sous un rayonnement UV/visible, à une longueur d'onde comprise entre 190 et 800 nm, de préférence sous un rayonnement UV de longueur d'onde comprise entre 300 et 380 nm.

La photopolymérisation dans l'étape iv) peut être réalisée pendant 1 à 10 heures, de préférence de 1 heure et 30 minutes à 3 heures.

Les différentes solutions sont préparées dans du tampon Tris ou HEPES, de préférence Tris-HCl, à une concentration de 25 à 100 mM, de préférence 50mM, à un pH de 7,2 à 7,5, de préférence 7,4 contenant du $CaCl_2$ de 10 à 50 mM, de préférence 20 mM et du NaCl de 50 à 250 mM, de préférence 150 mM.

Les concentrations finales des différents composés dans le mélange (étape ii) peuvent être par exemple :

- pour le fibrinogène de 1 à 50 mg/mL, de préférence entre 5 et 25 mg/mL,
- pour les monomères de formule (I) et éventuellement (II) de 1 à 400 mg/mL, de préférence 100 mg/mL,
- pour l'amorceur de 0,1 à 1 mg/mL, de préférence 0,42 mg/mL, et
- pour la thrombine de 0,1 à 2,5 U/mL, de préférence de 0,2 à 1 U/mL.

Après l'étape (iv), le procédé de l'invention peut, en outre, comprendre une étape de réticulation enzymatique du gel physique de fibrine. Dans cette étape supplémentaire, on peut utiliser par exemple la transglutaminase ou la lysyloxydase comme enzyme.

La transglutaminase est une enzyme qui ponte par liaisons covalentes (ou relie) certains acides aminés entre eux (essentiellement l'acide glutamique et la lysine) et qui contribue à la réticulation des protéines lors des processus de gélification. Cela peut ainsi conduire à un système plus résistant à la biodestruction et ayant des propriétés mécaniques améliorées.

Selon ce mode de réalisation, l'enzyme, par exemple la transglutaminase, peut être utilisée. L'échantillon de RIP obtenu après l'étape (iv) est alors plongé immédiatement ou ultérieurement dans une solution de transglutaminase à une concentration variant de 0,1 à 10 U/mL, de préférence entre 0,5 et 3 U/mL pendant des temps variables de 5 minutes à 6 heures, de préférence de 30 minutes à 3 heures.

Le procédé de l'invention peut être mis en oeuvre dans tout container, bécher, cristallisoir, pilulier, etc. pouvant de préférence être fermé. Le container peut également être réalisé sur mesure avec, par exemple, deux plaques de verre ou de tout matériau transparent aux UV et imperméable à l'eau. Ces plaques peuvent être séparées par un joint en Téflon□ ou en polyéthylène de différentes épaisseurs de 100 □m à 3 mm, de préférence d'1 mm.

L'invention concerne également le matériau sous forme de réseau interpénétré de polymères (RIP) associant un gel physique de fibrine et un réseau de polyéthylène glycol (PEG) susceptible d'être obtenu par le procédé selon l'invention.

Le matériau susceptible d'être obtenu par le procédé selon l'invention présente l'avantage d'être sous forme d'un gel, homogène et manipulable tout en présentant une stabilité dimensionnelle dans le temps et capable de résister à la dégradation par exemple par action de la chaleur. De plus, la résistance à la dégradation dudit matériau sous l'action des enzymes est significativement améliorée.

Comme indiqué précédemment, le matériau de l'invention est sous forme de réseau interpénétré de polymères (RIP) associant un gel physique de fibrine et un réseau de polyéthylène glycol (PEG) entre lesquels il n'y a aucun lien chimique, c'est-à-dire aucune liaison covalente. Dans ce réseau interpénétré de polymères (RIP), chaque polymère forme un réseau chimique ou physique ayant une existence tridimensionnelle ou 3D. Par exemple, le réseau PEG peut être synthétisé par réticulation de poly(éthylène glycol) di(méth)acrylate ($X_1 = X_2$ = (méth)acrylate où n peut varier de 2 à 140). Le réseau PEG gonfle dans l'eau ou dans le tampon.

Selon un mode de réalisation avantageux de l'invention, dans le matériau, le réseau interpénétré de polymères (RIP) associe un gel physique de fibrine et un réseau de polyéthylène glycol élaboré avec des poly(éthylène glycol) diméthacrylate ($X_1 = X_2$ = méthacrylate et n = 8 à 9 ou n = 13 à 14).

Selon un autre mode de réalisation avantageux de l'invention, il est possible de synthétiser le réseau de polyéthylène glycol avec des poly(éthylène glycol) diacrylate ($X_1 = X_2$ = acrylate et n = 8 à 9 ou n = 13 à 14).

Dans le matériau, le réseau de polyéthylène glycol (PEG) peut contenir entre 1 et 100% de monomères de formule (I) et entre 99 et 0% en masse de monomères de formule (II) tels que définis précédemment.

Plus particulièrement, les monomères de formule (I) sont ceux dans lesquels $X_1$ et $X_2$, identiques ou différents, sont utilisés seuls et sont choisis dans le groupe comprenant des méthacrylates ou acrylates et n est un nombre compris entre 2 et 20.

Le matériau de l'invention peut être constitué :

a) de 1 à 50 % en masse sèche de fibrine ;

b) de 50 à 99 % en masse sèche d'un réseau de polyéthylène glycol (PEG).

Le matériau selon l'invention présente des propriétés mécaniques améliorées par rapport à celles d'un gel constitué uniquement de fibrine.

Il possède aussi des propriétés (quasi-) réversibles de déshydratation et d'hydratation. Ceci n'est pas par exemple le cas pour le gel de fibrine seul. En effet, après un cycle de déshydratation, le gel de fibrine ne peut pas se réhydrater à plus de 25% de la valeur initiale.

Le matériau selon l'invention peut se réhydrater de 25 à 100%, de préférence de 45 à 95%.

Par ailleurs, le matériau selon l'invention a une résistance significativement améliorée à la dégradation, par exemple : par des protéases, par la température, etc. La fibrine est stabilisée dans ce matériau. En effet, lorsqu'un gel de fibrine est immergé dans du tampon Tris (50 mM pH 7,4) pendant 48h, 25% de la protéine est extraite. Dans le RIP cette proportion est comprise entre 20 et 0,1% généralement entre 1 et 0,1%. Le réseau de polyéthylène glycol (PEG) semble « protéger » la fibrine de la dégradation.

Le matériau selon l'invention présente une température de transition vitreuse (Tg) comprise entre -100 et +100°C de préférence entre -50 et +20°C. La température de transition vitreuse Tg (glass transition temperature en anglais) d'un matériau polymère se définit comme la température en-dessous de laquelle les chaînes polymère ont une faible mobilité relative. En dessous de Tg le polymère est dans un état vitreux, au-dessus de Tg, le polymère est dans un état caoutchoutique. La température de transition vitreuse d'un matériau peut être mesurée, par exemple, par l'analyse calorimétrique différentielle DSC.

Le matériau de l'invention peut présenter également un module de conservation (mesuré en mode cisaillement, G') compris entre 100 Pa et 10 MPa, par exemple entre 1000 Pa et 6 MPa, à l'état hydraté à 37°C et un module de conservation (mesuré en mode tension E') compris entre 0,01 et 3 000 MPa, par exemple entre 1 et 3000 MPa, à l'état sec.

Le module X d'un matériau soumis à une contrainte sinusoïdale s'écrit sous la forme complexe :

$$X^* = X' + iX''$$

Selon le mode de sollicitation utilisé, tension ou cisaillement, les modules X sont notés respectivement E ou G, X' sont notés E' ou G' et X " sont notés E" ou G". i est un nombre complexe tel que (i)□=1 [7].

En mode *tension,* la partie réelle du module E', *module élastique ou de conservation,* mesure la capacité du matériau à stocker de l'énergie alors que la partie imaginaire E", *module visqueux ou de perte,* représente la capacité du matériau à dissiper de l'énergie. Suivant l'état dans lequel se trouve le matériau en fonction de la température, un déphasage □ apparaît entre la contrainte exercée sur le matériau et sa déformation. Ce déphasage □ est nul lorsque le matériau est purement élastique, et de 90° lorsqu'il est parfaitement visqueux. Ainsi, la tangente de l'angle de déphasage (tan □) traduit la capacité d'amortissement du matériau.

Pour caractériser un matériau, les spectres de ATMD (analyse thermomécanique dynamique) représentant E' ou tan□ en fonction de la température sont enregistrés.

En *mode cisaillement,* le module de conservation ou d'élasticité en cisaillement (G') permet d'estimer l'élasticité du gel. Le module de perte ou module visqueux (G") caractérise la phase liquide du gel. Lorsque G">G', l'échantillon est considéré comme liquide et lorsque G'>G", l'échantillon est gélifié. Ainsi, les processus de gélification sont caractérisés par un temps de gel se traduisant par une égalité des modules G' et G".

Le matériau susceptible d'être obtenu selon le procédé de l'invention est avantageusement biocompatible. Lorsque l'on souhaite obtenir un matériau final stérile en sortie de synthèse (à l'issue de la mise en oeuvre du procédé de l'invention), le matériau est avantageusement obtenu par une photopolymérisation.

Un autre objet de l'invention concerne l'utilisation d'un matériau selon l'invention ou susceptible d'être obtenu selon le procédé de l'invention, comme :

- pansement pour les plaies,
- pansement chirurgical,
- un dispositif pour délivrer des agents thérapeutiques,
- revêtement pour des dispositifs médicaux choisi dans le groupe comprenant des stents, des valves pour le coeur, des cathéters, des filtres prosthétique vasculaires,
- support de molécules actives choisi dans le groupe comprenant des facteurs de croissance, des antibiotiques, bactéricides, bactériostatiques ou des enzymes,
- ou pour des cultures de cellules eucaryotes.

D'autres avantages pourront encore apparaître à l'homme du métier à la lecture des exemples ci-dessous, illustrés par les figures annexées, donnés à titre illustratif.

**Brève description des figures**

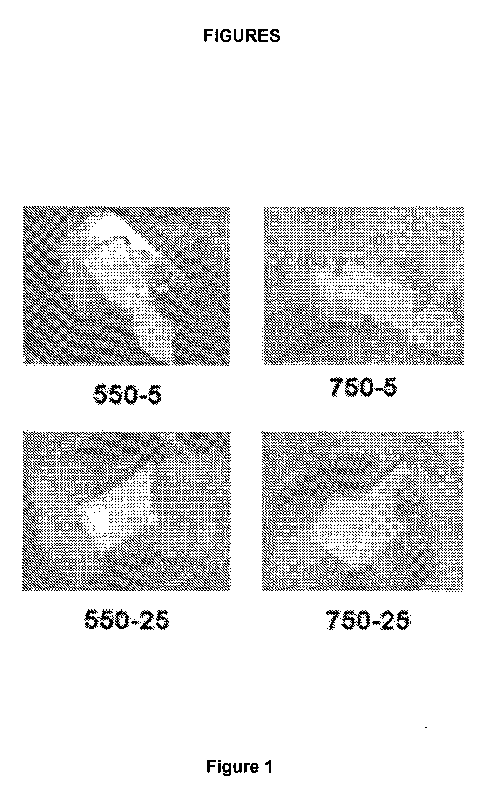

□ La figure 1 représente les photos juste après juste après l'étape iv) des RIPs obtenus à partir de mélanges de monomères (à la concentration finale 100mg/mL) de formule $X_1$-$(CH_2$-$CH_2$-$O)_n$-$X_2$ dans lesquelles $X_1$ et $X_2$ sont des groupements méthacrylate et :

- où n = 8 à 9 et le fibrinogène est à la concentration finale 5 mg/mL (RIP550-5)
- où n où n = 13 à 14 et le fibrinogène est à la concentration finale 5 mg/mL (RIP750-5)
- où n = 8 à 9 et le fibrinogène est à la concentration finale 25 mg/mL (RIP550-25)
- où n = 13 à 14 et le fibrinogène est à la concentration finale 25 mg/mL (RIP750-25).

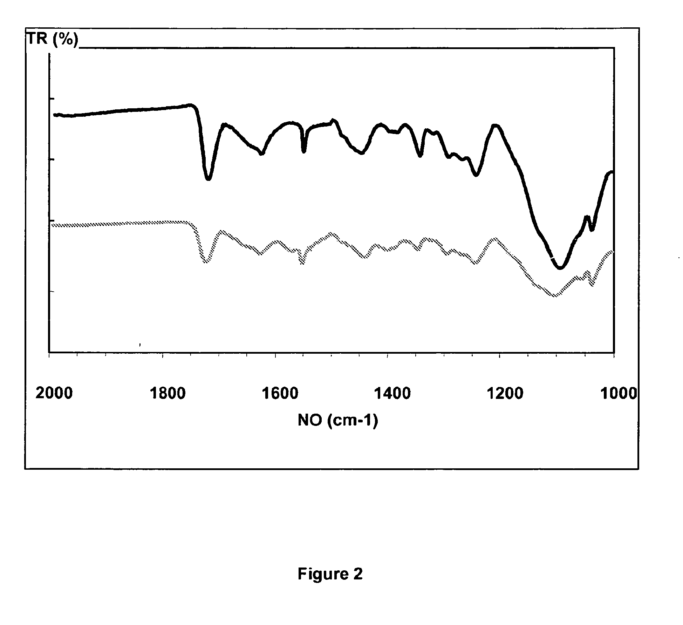

□ La figure 2 représente les spectres d'absorption mesurés avec un spectromètre infra-rouge à transformée de Fourier muni d'un module de réflexion totale atténuée (ATR) du RIP 550-5 (a) et du RIP 750/5 (b). En abscisse « NO » représente « Nombre d'Onde » et en ordonnée « TR » représente « Transmittance ».

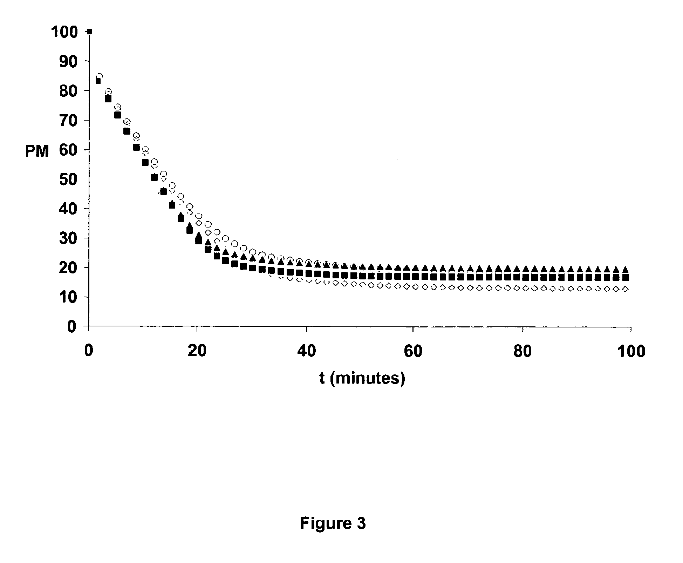

□ La figure 3 représente l'isotherme de déshydratation des différents RIPs mesurés avec un Analyseur Thermogravimétrique (ATG) sous flux d'argon à 37°C. (□: RIP 550-5; □ : RIP 550-25; □: RIP 750-5; □ : RIP 750-25). En abscisse « t » représente « temps » et en ordonnée « PM » représente « Perte de Masse ».

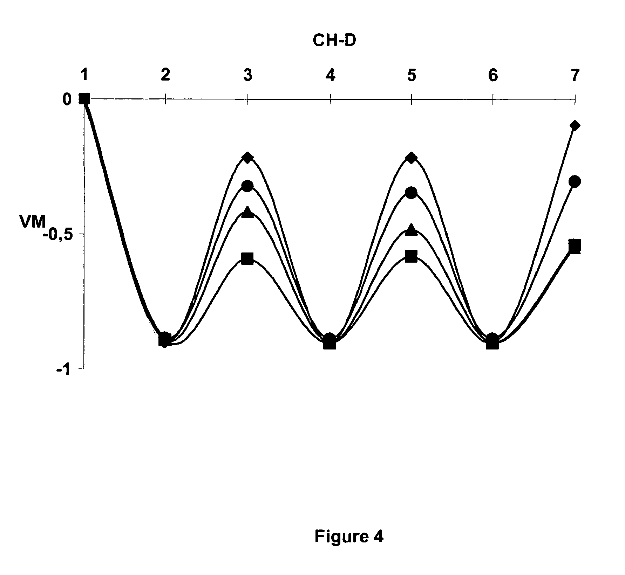

□ La figure 4 représente les cycles d'hydratation - déshydratation des RIPs 550-5 (□), RIP 550-25 (□), RIP 750-5 (□) et RIP 750-25 (□). En abscisse « CG-S » représente « Cycles de Gonflement-Séchage » et en ordonnée « VM » représente « Variation de Masse ».

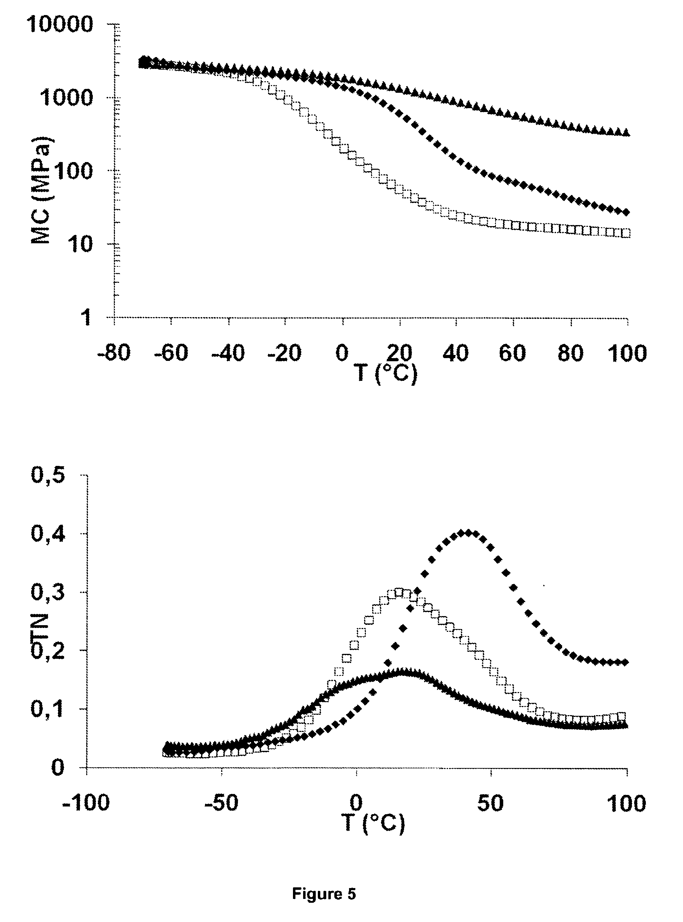

☐ La figure 5 représente l'analyse thermomécanique dynamique (ATMD) en mode tension en fonction de la température du réseau simple POE 550 (synthétisé dans les mêmes conditions sans fibrinogène dans le mélange (i), du RIP 550-5 et du RIP 550-25 séchés.

a- Modules de conservation (MC) ; de bas en haut : RIP 550-5, réseau simple POE 550, RIP 550-25. En ordonnée « T» représente « Température».

b- Tan Delta (TN) ; de haut en bas : réseau POE 550, RIP 550-5, RIP 550-25. En ordonnée « T» représente « Température».

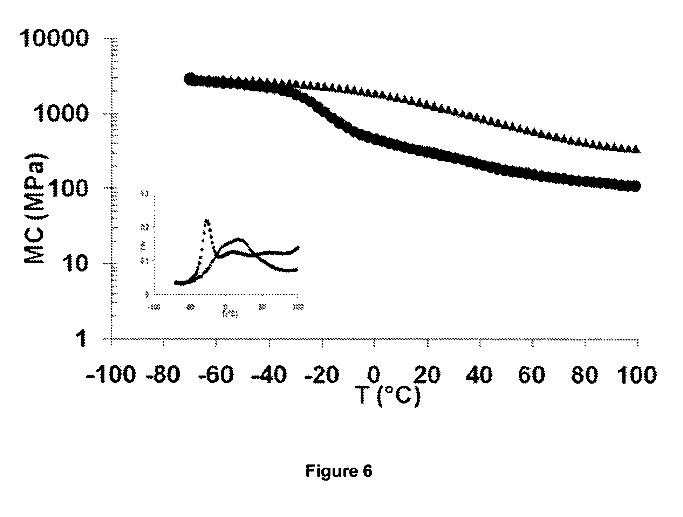

☐ La Figure 6 représente les modules de conservation en tension (MC) en fonction de la température (T) du RIP 550-25 (en haut) et du RIP 750-25 (en bas) - Dans la figure insérée : Tan Delta - de haut en bas : RIP 750-25, RIP 550-25.

## EXEMPLES

### I. Caractérisation des matériaux

### I.A. Caractérisations des propriétés mécaniques par analyse thermomécanique dynamique (ATMD)

Les caractérisations thermomécaniques en mode tension des différents matériaux (préalablement séchés 24h à 37°C dans une étuve) sont mesurées par ATMD sur un appareil Q800 (TA Instruments), avec une sollicitation en mode tension. La déformation imposée est de 0,05% (faible déformation réversible), la force de pré-tension de 120%. Les échantillons sont sollicités à une fréquence constante de 1Hz avec une rampe de température de T= - 80°C à 100°C à une vitesse de chauffe de 5°C/min. Les échantillons rectangulaires ont des tailles de longueur et de largeur variables mais une épaisseur moyenne de 0,1mm.

### I.B. Analyses thermiques

Les mesures d'analyse calorimétrique différentielle à balayage (DSC) ont été réalisées avec un appareil DSC Q100 (TA Instruments) et le logiciel d'analyse est Universal Analysis. L'échantillon séché d'une masse inférieure à 10 mg est déposé dans une capsule en aluminium qui est scellée, et placée dans le four. L'échantillon est refroidi à 5°C/min jusqu'à -80°C puis un isotherme d'une minute effectuée. Ensuite, une chauffe à 5°C/min est réalisée jusqu'à 200°C (premier balayage), suivie à nouveau d'un isotherme d'une minute. Un second passage est réalisé sur le même échantillon. Les thermogrammes enregistrés au second passage sont analysés pour déterminer les températures de transition vitreuses.

### I.C. Comportement hydrique des matériaux

### I.C.1. Isothermes de déshydratation

Les analyses thermogravimétriques (ATG) sont réalisées sur un Q50 model de la société TA Instruments et le logiciel d'analyse est Universal Analysis. Le changement de masse d'un échantillon est mesuré en fonction de la température et du temps sous une atmosphère d'argon.

Une masse m d'échantillon hydraté est déposée dans la nacelle, puis le four est fermé. Lors des enregistrements des isothermes, la température est équilibrée à 37°C, puis maintenue à cette température pendant 100 minutes, sous une atmosphère d'argon (60 mL.min$^{-1}$).

### I.C.2. Cycles d'hydratation-déshydratation

Les échantillons juste après l'étape (iv), sont plongés pendant 2 heures dans 25 mL de tampon Tris-HCl pH 7,4 contenant 0,02% d'azoture de sodium ($NaN_3$) afin d'atteindre le taux de gonflement maximum qui correspondra à une masse $m_1$ au point T1. Ensuite, les échantillons subissent 3 cycles alternés de déshydratation - hydratation. Les expériences de déshydratation se font pendant 24 heures à 37°C dans une étuve (masse $m_2$, $m_4$ et $m_6$ correspondant aux points T2, T4 et T6) et celles de réhydratation se font dans du tampon Tris pendant 2 heures à la même température (masse $m_3$ et $m_5$ correspondant aux points T3 et T5). La dernière étape amenant à la masse $m_7$ (point T7) est une réhydratation dans le tampon Tris-HCl de 24h à 37°C.

A partir des masses obtenues à chaque étape Ti, les courbes d'hydratation - déshydratation sont tracées en représentant la variation relative de masse $\Delta m = \dfrac{m_1 - m_i}{m_1}$ en fonction du numéro de l'étape réalisée.

### I.D. Analyses spectroscopiques

Les mesures d'absorbance sont réalisées sur un spectrophotomètre UVIKON XS double faisceau provenant de la société SECOMAM.

Les spectres d'infra rouge sont enregistrés sur un spectrophotomètre IR Tensor 27 équipé d'un module en réflexion ATR (cône diamant d'angle 45°).

### I.E. Tests ELISA

Les tests ELISA, permettant de quantifier la quantité de protéine extraite des matériaux, sont réalisés comme suit : les extractions sont faites en immergeant un échantillon de 1mL de gel de fibrine ou de RIP dans 4 mL de tampon de recouvrement une nuit à 37°C. La solution contenant les extractibles est diluée 100 fois (à l'exception des post-polymérisations avec la transglutaminase) avant le recouvrement des puits. Pour chaque résultat l'expérience est réalisée en n = 3 et au moins 3 fois.

Les tampons utilisés sont à base de tampon TBS (tampon salin Tris) composé de Tris 110 mM et NaCl 1,65 M à pH 7,4. Le tampon de fixation est composé de 10 mM de Tris, 150 mM de NaCl et 0,02% de $NaN_3$. Le tampon de saturation est composé de TBS contenant 5% de lait en poudre et 0,1% de Tween-20. Le tampon de rinçage et de dilution est composé de TBS contenant 0,05% de Tween-20 et 0,02% de $NaN_3$. Enfin, le pNPP est dissous dans 1 M de diéthanolamine contenant 0,5 mM de $MgCl_2$ à pH 9,8. La première étape du test ELISA consiste à fixer l'antigène en incubant 100 $\mu$L de l'extractible dans chacun des puits pendant 3 heures à 37°C. Après trois rinçages avec 200 $\mu$L de tampon de rinçage, 200 $\mu$L de tampon de saturation sont ajoutés dans les puits, le tout est incubé une nuit à 4°C. Après quatre rinçages avec 200 $\mu$L de tampon de rinçage, 100 $\mu$L de l'anticorps primaire dilué au 1/200 ème (anti-fibrinogène humain produit chez le lapin) sont introduits dans les puits et le tout est incubé pendant 3 heures à 37°C. Après rinçage, 100 $\mu$L de l'anticorps secondaire dilué au 1/30 000 ème (anti-immunoglobuline G de lapin produit chez la chèvre et couplé à la phosphatase alcaline) sont ajoutés, le tout est incubé pendant 3 heures à 37°C. Après rinçage, 200 $\mu$L de pNPP à 1 mg/mL (le substrat chromogène) sont ajoutés. Le développement de la coloration se fait à l'obscurité pendant 15 minutes et est stoppé par 50 $\mu$L de NaOH 3M. La mesure du dosage colorimétrique est réalisée à 405 nm, l'intensité du signal est directement proportionnelle à la concentration en fibrinogène.

### I.F. Extraction solide - liquide

L'extraction au dichlorométhane dans un soxhlet permet de solubiliser tous les composés solubles dans ce solvant (donc non réticulés) et de quantifier leur proportion dans un échantillon solide.

La quantité de matière soluble contenue dans les RIPs correspond à la proportion de monomère PEGDM non réticulé. Les matériaux sont extraits au soxhlet avec du $CH_2Cl_2$ à reflux pendant 48 heures. Les taux d'extractible sont calculés selon le rapport des masses : $Mat.Ext.\% = \dfrac{(m_i - m_f)}{m_i} x100$, où $m_i$ et $m_f$ sont respectivement les masses des matériaux avant et après extraction. Les extractions ont été réalisées sur des échantillons contenant 100 mg de réseau POE, c'est-à-dire que $m_i$=100mg.

### II. Caractéristiques des composés utilisés

Le chlorure de sodium (NaCl - CAS 27 800.291 Research organics), le dichlorure de calcium ($CaCl_2$, $2H_2O$ - CAS 22 317.297 - Research organics), l'acide chlorhydrique (37% HCl - CAS 20 252.290- Prolabo), l'urée (CAS 28876.367 - Prolabo), le dodécyl sulfate de sodium (SDS-CAS 16103.01 - Biorad), le $\square$-Mercaptoéthanol (CAS 16107.10 - Biorad), Le Diethanolamine ($C_4H_{11}NO_2$ - CAS 111-42-2), le Tween-20 Polyoxyethylene-Sorbitan Monolaurate (CAS 9005-64-5) et l'azodure de sodium ($NaN_3$ - CAS 26628-22-8) proviennent de la société Sigma. Les différents poly(oxyde d'éthylène)diméthacrylate (PEGDM - CAS 25852-47-5 - Aldrich) de masses molaires 330 (PEGDM330), 550 (PEGDM550) et 750 (PEGDM750) g.mol$^{-1}$, le poly(éthylène glycol) méthacrylate monofonctionnel (530 g.mol$^{-1}$ - ALDRICH), le 2-hydroxy-4-(2-hydroxyethoxy)-2-methylpropiophenone (Irgacure 2959 - CIBA), le dichlorométhane (Carlo-Erba), trihydroxyaminométhane (Tris, Prolabo ou Carlo Erba), sont utilisés sans traitement préalable.

Le p-Nitrophenyl Phosphate (pNPP) et l'iodoacétamide (I-6185 - CAS 144-48-9) proviennent de la société SIGMA. Ce substrat chromogène (EC 224-246-5) est en tablette et sous forme de comprimés de 5mg.

Le Fibrinogène type IV (Fg, F-4753 - SIGMA) utilisé est d'origine bovine. La protéine lyophilisée est conservée à -20°C et est gardée à cette même température après solubilisation.

La Thrombine lyophilisée (EC3.421.5 - 34.8unité par mg de solide-SIGMA) est conservée à -20°C. Sa solution dans le tampon Tris-HCl peut être stockée 2 à 3 jours à +4°C et -20°C pour une durée plus longue.

La Transglutaminase (Activa WM - société Ajinomoto distribuée par UNIPEX), conditionnée en poudre, a une activité de 100 unités par gramme de solide et est conservée à -20°C.

La Thermolysine (SIGMA) possède 33 unités par mg de solide et est conservée à -20°C.

Pour les expériences d'ELISA, l'anticorps primaire anti-fibrinogène humain produit chez le lapin provient de la société DAKO (CAS - A 0080). L'anticorps secondaire anti-IgG (Immunoglobuline G - A 3687 - Sigma) de lapin est produit chez la chèvre et couplé à la phosphatase alcaline.

Toutes les solutions sont préparées dans du tampon Tris-HCl 50 mM pH 7,4. Des solutions mères à 2 M $CaCl_2$, 1,5 M NaCl ainsi que 10 mg/mL et 50 mg/mL de fibrinogène sont préparées. Les solutions mères de thrombine et transglutaminase sont respectivement préparées à 20 unités/mL et 10 unités /mL dans le tampon tris-HCl.

## III. Exemples d'élaborations de matériaux et leur caractérisation

### Exemple 1 : Synthèse du RIP POE550 / Fibrine (100/5) (RIP550-25)

Les solutions mères de fibrinogène (10 mg/mL), de thrombine (20 U/mL), de NaCl (1,5 M), de $CaCl_2$ (2 M), et d'Irgacure 2959 (6 mg/mL) préparées dans du tampon Tris-HCl (50mM, pH 7,4) ainsi que le monomère PEGDM550 pur sont incubés séparément pendant 15 minutes à 37°C dans un bain marie.

210 $\mu$L de tampon Tris-HCl, 10 $\mu$L de $CaCl_2$ (2 M), 100 $\mu$L de NaCl (1,5 M), 500 $\mu$L de fibrinogène (10 mg/mL), 100 $\mu$L de monomère PEGDM 550 et 70 $\mu$L d'Irgacure 2959 (6 mg/mL) sont introduits dans cet ordre dans un tube ependorff de 1,5 mL. La réaction de gélification de la fibrine est initiée par l'addition de 10 $\mu$L d'une solution de thrombine dans le tampon Tris-HCl à la concentration de 20 unités/mL. Le mélange réactionnel d'un volume total de 1 mL est homogénéisé à la pipette et rapidement introduit entre deux lames de verre séparées par un joint en Téflon (marque enregistrée) d'une épaisseur moyenne de 1 mm. Ces lames tenues par des pinces sont déposées sur un support se trouvant dans un bain marie maintenu à 37°C. La polymérisation radicalaire est réalisée pendant 1h30min à l'abri de la lumière du jour avec une lampe de type VL-6 ayant une puissance de 2□5W émettant des rayonnements UV à □= 365 nm.

Le RIP 550-5 est un matériau mou déformable se présentant sous la forme d'un rectangle de 6,5 cm x 2 cm et d'une épaisseur moyenne de 1 mm (Figure 1).

Le RIP 550-5 contient une fraction soluble au dichlorométhane de 36% en masse par rapport à la masse de monomère PEGDM550 introduite initialement. 10% de la masse de protéine peut être extraite de ce RIP. Le spectre infra-rouge du RIP est reporté (Figure 2). Ses comportements à la déshydratation sous flux d'argon à 37°C (mesuré par ATG), d'une part, et aux cycles d'hydratation-déshydratation, d'autre part, sont reportés, respectivement, (Figure 3 et Figure 4).

Le RIP 550-5 présente une température de transition vitreuse (Tg) détectée à -37°C par mesure DSC lors du second balayage.

Le RIP 550-5 présente un module de conservation en mode cisaillement de l'ordre de 0,8 MPa à l'état hydraté (mesuré en ATMD en mode cisaillement). Les propriétés thermomécaniques de ce RIP à l'état séché sont reportées (Figure 5).

### Exemple 2 : Synthèse du RIP POE550 / Fibrine (100/25) (RIP 550-25)

Le RIP 550-25 (Figure 1) est synthétisé suivant le même protocole que celui décrit dans l'Exemple 1. Seule la concentration de la solution mère de fibrinogène est fixée à 25 mg/mL, toutes les autres concentrations restent identiques. Les volumes introduits sont les mêmes.

Le RIP POE550 / Fibrine (100/25) ne contient pas de fraction soluble au dichlorométhane (0% d'extractible au dichlorométhane). Les tests ELISA montrent que 1% en masse de protéine introduite est extrait du RIP. Ce RIP présente une température de transition vitreuse (Tg) détectée par DSC à -27°C. Ses comportements à la déshydratation sous flux d'argon à 37°C (mesuré par ATG), d'une part, et aux cycles d'hydratation-déshydratation, d'autre part, sont reportés, respectivement, (Figure 3 et Figure 4).

Le RIP 550-25 présente un module de cisaillement de l'ordre de 0,3 MPa à l'état hydraté (mesuré en ATMD en mode cisaillement). Les propriétés thermomécaniques de ce RIP à l'état séché sont reportées (Figure 5 et Figure 6).

**Exemple 3 : Synthèse du RIP POE750 / Fibrine (100/5) (RIP 750-5)**

Le RIP 750-5 est synthétisé suivant le même protocole que celui décrit dans l'Exemple 1. Seul le monomère PEGDM 550 est remplacé par le monomère PEGDM 750. Les concentrations et les volumes introduits sont les mêmes.

Le RIP 750-5 est rigide et cassant : il est possible de le découper avec une embout de pipette (Figure 1). Il se craquelle au cours du séchage. Il contient une fraction soluble au dichlorométhane de 24% en masse par rapport à la masse de monomère PEGDM750 introduite. 25% de la masse de protéine peut être extraite de ce RIP. Ce RIP présente une température de transition vitreuse (Tg) détectée par DSC à -38°C. Le spectre infra-rouge du RIP est reporté (Figure 2). Ses comportements à la déshydratation sous flux d'argon à 37°C (mesuré par ATG), d'une part, et aux cycles d'hydratation-déshydratation, d'autre part, sont reportés, respectivement (Figure 3 et Figure 4).

**Exemple 4 : Synthèse du RIP POE750 / Fibrine (100/25) (RIP 750-25)**

Le RIP 750-25 (figure 1) est synthétisé suivant le même protocole que celui décrit dans l'Exemple 2. Seul le monomère PEGDM 550 est remplacé par le monomère PEGDM 750. Les concentrations et les volumes introduits sont les mêmes.

Le RIP 750-25 contient 0% de fraction soluble au dichlorométhane. Les tests ELISA montrent que 1% en masse de protéine introduite est extrait du RIP. Ses comportements à la déshydratation sous flux d'argon à 37°C (mesuré par ATG), d'une part, et aux cycles d'hydratation-déshydratation, d'autre part, sont reportés, respectivement (Figure 3 et Figure 4).

Le RIP 750-25 présente un module de cisaillement de l'ordre de 1,2 MPa à l'état hydraté (mesuré en ATMD en mode cisaillement). Les propriétés thermomécaniques de ce RIP à l'état séché sont reportées (Figure 6).

**Exemple 5 : Post-réticulation enzymatique du réseau de fibrine dans le RIP POE550 / Fibrine (100/5) (RIP 550-5)**

Le RIP 550-5 synthétisé suivant le protocole décrit dans l'Exemple 1 est plongé pendant 3 heures dans 10 mL d'une solution de transglutaminase à 0,5 unité/mL dans le tampon Tris-HCl. Les tests ELISA montrent que moins de 0,2% de la masse de protéine introduite peut être extraite de ce RIP.

**Exemple 6 : Post-réticulation enzymatique du réseau de fibrine dans le RIP POE550 / Fibrine (100/25) (RIP 550-25)**

Le RIP 550-25 synthétisé suivant le protocole décrit dans l'Exemple 2 est plongé pendant 3 heures dans 10 mL d'une solution de transglutaminase à 0,5 unité/mL dans le tampon Tris-HCl. Les tests ELISA montrent que 0,2% de la masse de protéine introduite peut être extraite de ce RIP. Ce matériau présente un module de cisaillement de l'ordre de 1 MPa à l'état hydraté (mesuré en ATMD en mode cisaillement à 1 Hz).

**Exemple 7 : Post-réticulation enzymatique du réseau de fibrine dans le RIP POE750 / Fibrine (100/5) (RIP 750-5)**

Le RIP 750-5 synthétisé suivant le protocole décrit dans l'Exemple 3 est plongé pendant 3 heures dans 10 mL d'une solution de transglutaminase à 0,5 unité/mL dans le tampon Tris-HCl. Les tests ELISA montrent que 0,2% de la masse de protéine introduite peut être extraite de ce RIP.

**Exemple 8 : Post-réticulation enzymatique du réseau de fibrine dans le RIP POE750 / Fibrine (100/25) (RIP 750-25)**

Le RIP 750-25 synthétisé suivant le protocole décrit dans l'Exemple 4 est plongé pendant 3 heures dans 10 mL d'une solution de transglutaminase à 0,5 unité/mL dans le tampon Tris-HCl. Les tests ELISA montrent que 0,2% de la masse de protéine introduite peut être extraite de ce RIP. Ce matériau présente un module de cisaillement de l'ordre de 3 MPa à l'état hydraté (mesuré en ATMD en mode cisaillement à 1 Hz).

**Exemple 9 : Biocompatibilité des RIP**

Des cellules CHOK1 ont été mises en culture sur les RIPs 750-5 et RIP 750/25 décrits, respectivement dans l'exemple 3 et l'exemple 4. Pour les deux matériaux:

- les cellules adhèrent aux matériaux, en particulier sur le RIP 750/25.
- un marquage des noyaux au DAPI (4',6-diamino-2-phényl-indole) montre que les cellules sont vivantes après au moins 5 jours.
- une observation en microcopie à fluorescence montre que les cellules se divisent après avoir adhéré sur le matériau

- un marquage fluorescent du réseau d'actine prouve que les cellules s'étalent sur les matériaux.
- plusieurs cellules ont pu être observées en phase de mitose. Les cellules se multiplient sur les matériaux.
- les cellules ont tendance à pénétrer dans le matériau en particulier pour le RIP 750/25

Toutes ces observations permettent de conclure que les RIP 750-5 et RIP 750/25 sont biocompatibles.

**Exemple 10 : Capacité de rétention / relargage de molécules**

Le RIP 750/25 décrit dans l'exemple 4, a été rincé abondamment en sortie de synthèse, puis immergé dans un milieu de culture adapté aux CHOK1, séché par du papier absorbant, puis ensemencé en cellules eucaryotes. Les cellules adhèrent et présentent une activité cellulaire plus grande sur ce matériau que sur le même matériau non "enrichi" décrit dans l'exemple 10.

**Exemple 11 : Capacité de rétention / relargage de molécules**

Le RIP 750/25 décrit dans l'exemple 4 est rincé avec du tampon Tris-HCl en sortie de synthèse, séché avec du papier absorbant, puis immergé dans une solution d'antibiotique et séché avec du papier absorbant. Trois antibiotiques différents : le chloramphénicol, la streptomycine et l'ampicilline ont été utilisés. Ces matériaux sont ensuite déposés sur une boîte de Pétri couverte de bactéries. Après 24 h, dans chaque cas, une zone abiotique (sans présence de vie) est observée montrant que l'antibiotique a été relargué par le matériau. Tous les contrôles où le matériau est seul sont négatifs.
Le RIP 750/25 présente donc des capacités de rétention / relargage de molécules principes actifs.

**Exemple 12 : Comparaison avec d'autres oligomères**

La faisabilité d'autres RIPs et leurs propriétés mécaniques ont été vérifiées en faisant tout d'abord varier les longueurs de chaînes de l'oligomère PEGDM, c'est-à-dire la densité de réticulation du réseau POE synthétisé dans le RIP.
Le PEGDM 330 avec une masse molaire plus faible (330 g.mol$^{-1}$) que le PEGDM 550 a été choisi. Une concentration de monomère synthétique de 100 mg/mL est conservée. Le PEGDM 330 n'est pas suffisamment soluble dans l'eau (solubilité : 65 mg/mL), sa solution à 100 mg/mL dans le tampon Tris-HCl n'est donc pas homogène et la synthèse du RIP aboutit logiquement à un matériau inhomogène.
Des réseaux POE synthétisés à partir de mélanges 10/90 et 90/10 en masse de PEGDM 550 ou PEGDM 750 avec du poly(éthylène glycol) méthacrylate monofonctionnel de masse 530 g.mol$^{-1}$ ont également été associés au gel de fibrine afin de faire varier la densité de réticulation du réseau POE. Une concentration totale en monomère poly(oxyde d'éthylène) de 100 mg/mL est conservée. Des matériaux de texture gel mou qui ne présentent pas de tenue mécanique acceptable ont été obtenus.

Listes **des références**

[1] Ruszczak, Z.. (2003) "Effect of collagen matrices on dermal wound healing". Advanced Drug Delivery Reviews 55 : 1595- 1611

[2] Nguyen, K. T. and J. L. West (2002). "Photopolymerizable hydrogels for tissue engineering applications." Biomaterials 23: 4307-4314.

[3] Schmedlen, R. H., J. L. Masters, et al. (2002). "Photocrosslinkable polyvinyl alcohol hydrogels that can be modified with cell adhesion peptides for use in tissue engineering." Biomaterials 23: 4325-4332.

[4] De Gennes, P.-G. (1979). Scaling Concerts in Polymer Physics. London, Cornell University Press.

[5] Verderio E. A. M., Johnson, T., Griffin, M.. (2004) " Tissue transglutaminase in normal and abnormal wound healing". Amino Acids 26: 387-404

[6] W. Bensaïd, Triffitt J. T., Blanchat, C. Oudina, K. Sedel, L. , Petite, H. (2003) "A biodegradable fibrin scaffold for mesenchymal stem cell transplantation", Biomaterials, 24: 2497-2502.

[7] M.Fontanille, Y.Gnanou "chimie et physico-chimie des polymères "Edition Dunod, (2002) ISBN 2-10-049493-7 page : 410-413.

**Revendications**

1. Procédé de préparation d'un matériau sous forme de réseau interpénétré de polymères (RIP) associant un gel physique de fibrine et un réseau de polyéthylène glycol (PEG), comprenant les étapes suivantes :

    i) préparer un mélange en introduisant dans du tampon

       - une solution de fibrinogène,
       - des monomères de formule (I) :

$$X_1\text{-}(CH_2\text{-}CH_2\text{-}O)_n\text{-}X2$$

       dans laquelle $X_1$ et $X_2$, sont des groupements chimiques identiques ou différents, choisis dans le groupe comprenant le vinyle, l'acrylate, le méthacrylate ou l'allyle et n est un nombre compris entre 1 et 140,
       - éventuellement des monomères de formule (II) choisis parmi les dérivés de polyéthylène glycol portant un groupement choisi dans le groupe comprenant $-CH_3$, $-NH_2$, $-OH$, $-CH_2CH_3$, le vinyle, l'acrylate, le méthacrylate ou l'allyle ; les dérivés d'acrylate, de méthacrylate et de styrène choisis dans le groupe comprenant l'hydroxyéthylméthacrylate, l'hydroxyéthylacrylate, l'acétate de vinyle, la N-vinyl pyrrolidone, la N-vinyl pyridine, l'acrylonitrile, l'acide acrylique, le styrène sulfonate de sodium, le bromure de (vinylbenzyl)triméthyl- ammonium, le chlorure de (vinylbenzyl)triméthyl-ammonium, le chlorure de triméthyl-vinyloxycarbonylméthyl-ammonium, le bromure de triméthyl-vinyloxycarbonylméthyl-ammonium, le bromure de triéthyl-2-vinyloxycarbonyl-éthyl)-ammonium, le bromure d'allyloxycarbonylméthyl-triméthyl ammonium, le poly(éthylène glycol) méthacrylate, le poly(éthylène glycol) acrylate,
       - un amorceur de polymérisation ;

    ii) préparer un mélange réactionnel en ajoutant une solution de thrombine au mélange préparé en i) ;
    iii) incuber le mélange réactionnel obtenu en ii) à une température comprise entre 20 et 40 °C ;
    iv) réaliser une polymérisation et réticulation des monomères de formule (I) avec éventuellement les monomères de formule (II).

2. Procédé selon la revendication 1, dans lequel le polymère de polyéthylène glycol (PEG) contient entre 1 et 100% en masse de monomères de formule (I) et entre 99 et 0% en masse de monomères de formule (II).

3. Procédé selon l'une des revendications 1 ou 2, dans lequel les monomères de formule (I) sont ceux dans lesquels $X_1$ et $X_2$, identiques ou différentes, choisis dans le groupe comprenant des méthacrylates ou acrylates et n est un nombre compris entre 2 et 20.

4. Procédé selon l'une quelconque des revendication 1 à 3, dans lequel l'amorceur de polymérisation est un amorceur de photopolymérisation.

5. Procédé selon la revendication 4, dans lequel l'amorceur de photopolymérisation est choisi dans le groupe comprenant les Irgacures (2959, 184, 651, 819), le 2-hydroxy-4-(2-hydroxyéthoxy)-2-méthylpropiophénone, la benzophénone, le 2, 4- diméthylbenzophénone, la benzoïne, les benzophénones ioniques choisis dans le groupe comprenant le chlorure de 4-triméthylméthylammonium benzophénone, le sel de sodium du 4-sulfométhylbenzyl, les cétones aromatiques et les aldéhydes notamment le benzaldéhyde, l'acétophénone, le biacétyle, le parachlorobenzophénone, l'acide ferrulique.

6. Procédé selon l'une quelconque des revendications 1 à 3, dans lequel l'amorceur de polymérisation est un amorceur thermique.

7. Procédé selon la revendication 6, dans lequel l'amorceur thermique est choisi dans le groupe comprenant les peroxydes, les composés diazoïques ou les persulfates.

8. Procédé selon l'une quelconque des revendications 1 à 7, dans lequel dans l'étape iii), la température d'incubation est de 37°C.

9.  Procédé selon l'une quelconque des revendications 1 à 5 et 8, dans lequel dans l'étape iv), la polymérisation est réalisée sous un rayonnement UV/visible à une longueur d'onde comprise entre 190 et 800 nm.

10.  Procédé selon la revendication 9, dans lequel dans l'étape iv), la polymérisation est réalisée pendant 1 à 10 heures.

11.  Procédé selon l'une quelconque de revendications 1 à 3 et 6 à 8, dans lequel la polymérisation a lieu en même temps que l'étape (iii).

12.  Procédé selon l'une quelconque de revendications 1 à 3 et 6 à 8, dans lequel la polymérisation a lieu après l'étape (iv), à une température comprise entre 25 et 60°C.

13.  Procédé selon l'une quelconque de revendications 1 à 12, dans lequel après l'étape (iv), le procédé de l'invention comprend en outre une étape de réticulation enzymatique du gel physique de fibrine.

14.  Procédé selon la revendication 13, dans lequel l'enzyme utilisée est la transglutaminase.

15.  Matériau sous forme de réseau interpénétré de polymères (RIP) associant un gel physique de fibrine et un réseau de polyéthylène glycol (PEG) susceptible d'être obtenu par le procédé selon l'une quelconque des revendications 1 à 14.

16.  Matériau selon la revendication 15, dans lequel le réseau de polyéthylène glycol (PEG) contient entre 1 et 100% en masse de monomères de formule (I) et entre 99 et 0 % de monomères de formule (II).

17.  Matériau selon l'une des revendications 15 ou 16, dans lequel les monomères de formule (I) sont ceux dans lesquels $X_1$ et $X_2$, sont des groupements chimiques identiques ou différents, choisis dans le groupe comprenant des méthacrylates ou acrylates et n est un nombre compris entre 2 et 20.

18.  Matériau sous forme de réseau interpénétré de polymères (RIP) selon l'une quelconque des revendications 15 à 17, constitué :

    a) de 1 à 50 % en masse sèche de fibrine ;
    b) de 50 à 99 % en masse sèche d'un réseau de polyéthylène glycol (PEG).

19.  Matériau selon l'une quelconque des revendications 15 à 18, présentant une température de transition vitreuse (Tg) comprise entre -100 et +100°C.

20.  Matériau selon l'une quelconque des revendications 15 à 19, présentant un module de cisaillement (mesuré en mode de cisaillement G') compris entre 100 Pa et 10 MPa à l'état hydraté à 37°C et un module de conservation (mesuré en mode tension E') compris entre 0 ,01 et 3 000 MPa à l'état sec.

21.  Utilisation d'un matériau selon l'une quelconque des revendications 15 à 20 ou susceptible d'être obtenu selon le procédé selon l'une quelconque des revendications 1 à 14, comme :

    - pansement pour les plaies,
    - pansement chirurgical,
    - un dispositif pour délivrer des agents thérapeutiques,
    - revêtement pour des dispositifs médicaux choisi dans le groupe comprenant des stents, des valves pour le coeur, des cathéters, des filtres prosthétiques vasculaires,
    - support de molécules actives choisi dans le groupe comprenant des facteurs de croissance, des antibiotiques, bactéricides, bactériostatiques ou des enzymes,
    - ou pour des cultures de cellules eucaryotes.

**Patentansprüche**

1.  Verfahren zur Herstellung eines Materials in Form eines interpenetrierenden Polymernetzwerks (IPN), das ein physisches Fibringel und ein Polyethylenglykolnetzwerk (PEG) kombiniert, umfassend folgende Schritte :

i) Herstellen eines Gemischs unter Einbringen von folgenden in einen Puffer:

- einer Fibrogenlösung,
- von Monomeren der Formel (I) :

$$X_1\text{-}(CH_2\text{-}CH_2\text{-}O)_n\text{-}X_2$$

wobei $X_1$ und $X_2$ identische oder unterschiedliche chemische Gruppen sind, die aus der Gruppe ausgewählt werden, die Vinyl, Acrylat, Methacrylat oder Allyl umfasst, und n eine Zahl zwischen 1 und 140 ist,
- gegebenenfalls von Monomeren der Formel (II) ausgewählt aus Polyethylenglykol-Derivaten mit einer funktionellen Gruppe ausgewählt aus der Gruppe umfassend $-CH_3$, $-NH_2$, $-OH$,$-CH_2CH_3$, Vinyl, Acrylat, Methacrylat oder Allyl; Derivate von Acrylat, Methacrylat und Styren ausgewählt aus der Gruppe umfassend Hydroxyethylmethacrylat, Hydroxyehtylacrylat, Vinylacetat, N-Vinylpyrrolidon, N-Vinylpyridin, Acrylonitril, Acrylsäure, Natrium-Styrolsulfonat, (Vinylbenzyl)- trimethylammoniumbromid, (Vinylbenzyl)-trimethyl-am-monium-chlorid, Trimethyl-vinyloxycarbonymethyl-ammoniumchlorid, Trimethyl-vinyloxycarbonymethyl-ammoniumbromid, Triethyl-(2-vinyloxycarbonyl-ethyl)-ammoniumbromid, Allyloxycarbonylmethyl-trimethyl-ammoniumbromid, Poly(ethyleneglykol)methacrylat, Poly(ethyleneglykol)acrylat, eines Polymerisationsinitiators ;

ii) Herstellen eines Reaktionsgemischs durch Zufügen einer Thrombinlösung zu der in i) zubereiteten Mischung;
iii) Inkubation des in ii) erhaltenen Reaktionsgemischs bei einer Temperatur zwischen 20 und 40 °C ;
iv) Durchführen einer Polymerisation und Vernetzung der Monomere der Formel (I) gegebenenfalls mit Monomeren der Formel (II).

2. Verfahren nach Anspruch 1, wobei das Polyethylenglykol (PEG) 1 bis 100 Massen-% der Monomere der Formel (I) und 99 bis 0 Massen-% der Formel (II) enthält.

3. Verfahren nach einem der Ansprüche 1 oder 2, wobei die Monomere der Formel (I) jene sind, in denen $X_1$ und $X_2$ identisch oder unterschiedlich sind, ausgewählt aus der Gruppe umfassend Methacrylate oder Acrylate und n eine Zahl zwischen 2 und 20 ist.

4. Verfahren nach einem der Ansprüche 1 bis 3, wobei der Polymerisationsinitiator ein Fotopolymerisationsinitiator ist.

5. Verfahren nach Anspruch 4, wobei der Fotopolymerisationsinitiator aus der Gruppe umfassend Irgacure (2959, 184, 651, 819), 2-Hydroxy-4-(2-Hydroxyethoxy)-2-methylpropiophenon, Benzophenon, 2, 4-Dimethylbenzophenon, Benzoin, ionisches Benzophenon ausgewählt wird, ausgewählt aus der Gruppe umfassend 4-Trimethylmethylammonium-Benzophenonchlorid, Natriumsalz von 4-Sulfomethylbenzyl, aromatische Ketone und Aldehyde, insbesonders Benzaldehyd, Acetophenon, Biacetyl, Parachlorobenzophenon, Ferualsäure.

6. Verfahren nach einem der Ansprüche 1 bis 3, wobei der Polymerisationsinitiator ein thermischer Initiator ist.

7. Verfahren nach Anspruch 6, wobei der thermische Initiator aus der Gruppe ausgewählt wird, die Peroyxde, Diazoverbindungen oder Persulfate umfasst.

8. Verfahren nach einem der Ansprüche 1 bis 7, wobei in Schritt iii) die Inkubationstemperatur 37°C beträgt.

9. Verfahren nach einem der Ansprüche 1 bis 5 und 8, wobei in Schritt iv) die Polymerisation unter bei einer Wellenlänge zwischen 190 und 800 nm sichtbaren UV-Strahlung durchgeführt wird.

10. Verfahren nach Anspruch 9, wobei in Schritt iv) die Polymerisation während 1 bis 10 Stunden durchgeführt wird.

11. Verfahren nach einem der Ansprüche 1 bis 3 und 6 bis 8, wobei die Polymerisation gleichzeitig mit Schritt (iii) stattfindet.

12. Verfahren nach einem der Ansprüche 1 bis 3 und 6 bis 8, wobei die Polymerisation nach Schritt (iv) bei einer Temperatur zwischen 25 und 60°C stattfindet.

**13.** Verfahren nach einem der Ansprüche 1 bis 12, in welchem nach Schritt (iv) das erfindungsgemässe Verfahren ferner einen Schritt der enzymatischen Vernetzung des physischen Fibringels umfasst.

**14.** Verfahren nach Anspruch 13, wobei das verwendete Enzym Transglutaminase ist.

**15.** Material in Form eines interpenetrierenden Netzwerks (IPN), das ein Fibringel und ein Polyethylenglykolnetzwerk (PEG) kombiniert, das durch das Verfahren nach einem der Ansprüche 1 bis 14 erhalten werden kann.

**16.** Material nach Anspruch 15, in welchem das Polyethylenglykolnetzwerk (PEG) zwischen 1 et 100 Massen-% Monomere der Formel (I) und zwischen 99 und 0 Massen-% Monomere der Formel (II) enthält.

**17.** Material nach einem der Ansprüche 15 oder 16, in welchem die Monomere der Formel (I) jene sind, in denen $X_1$ und $X_2$ identische oder unterschiedliche chemische Gruppen sind, ausgewählt aus der Gruppe umfassend Methacrylate oder Acrylate und n eine Zahl zwischen 2 und 20 ist.

**18.** Material in Form von interpenetrierenden Netzwerken (IPN), nach einem der Ansprüche 15 bis 17 bestehend aus :

    a) 1 bis 50 Trockenmassen-% Fibrin ;
    b) 50 bis 99 Trockenmassen-% eines Polyethylenglykolnetzwerks (PEG).

**19.** Material nach einem der Ansprüche 15 bis 18 mit einer Glasübergangstemperatur (Tg) zwischen -100 und +100°C.

**20.** Material nach einem der Ansprüche 15 bis 19 mit einem Schermodul (im Schermodus G' gemessen) zwischen 100 Pa und 10 MPa im befeuchteten Zustand bei 37°C und einem Speichermodul (im Spannungsmodus E' gemessen) zwischen 0,01 und 3 000 MPa im trockenen Zustand.

**21.** Verwendung eines Materials, das nach einem der Ansprüche 15 bis 20 oder mit einem Verfahren nach einem der Ansprüche 1 bis 14 erhalten werden kann, als :

    - Wundverband,
    - chirurgischer Verband,
    - Vorrichtung zur Verabreichung therapeutischer Mittel,
    - Beschichtung von medizinischen Vorrichtungen ausgewählt aus der Gruppe umfassend Stents, Herzklappen, Katheder, prosthetische Blutgefässfilter,
    - Unterstützung aktiver Moleküle ausgewählt aus der Gruppe umfassend Wachstumsfaktoren, Antibiotika, Bakterizide, Bakteriostatika oder Enzyme,
    - oder für die eukarozytische Zellkultur.

## Claims

**1.** A method of preparing a material in the form of interpenetrating polymers network (IPN) associating a physical gel of fibrin and a polyethylene glycol (PEG) network, comprising the following steps:

    i.preparing a mixture by introducing into a buffer

        - a fibrinogen solution
        - monomers of formula (I):

$$X_1\text{- } (CH_2\text{-}CH_2\text{-}O)n\text{-}X_2$$

        wherein $X_1$ and $X_2$ are identical or different chemical group selected from the group comprising vinyl, acrylate, methacrylate or allyl and n is a number between 1 and 140,
        - optionally monomers of formula (II) selected from polyethylene glycol derivatives having a group selected from the group comprising-$CH_3$, -$NH_2$, -OH, -$CH_2CH_3$, vinyl, acrylate, methacrylate or allyl; acrylate derivatives, methacrylate and styrene selected from the group comprising hydroxyethylmethacrylate, hydroxyethylacrylate, vinyl acetate, N-vinyl pyrrolidone, N-vinylpyridine, acrylonitrile, acrylic acid, sodium styrene sulfonate, (vinylbenzyl)trimethylammonium bromide, (vinylbenzyl)trimethylammonium chloride, trimethylvinyloxycarbonylmethyl-ammonium chloride, vinyloxycarbonylmethyl-ammonium bromide, triethyl-2-vinyl-loxycarbonyl-ethyl)-ammonium bromide, allyloxycarbonylmethyl-trimethyl ammonium bromide, poly(ethylene glycol) methacrylate, poly(ethylene glycol) acrylate,
- a polymerization initiator;

ii. preparing a reaction mixture by adding a thrombin solution to the mixture prepared in i);
iii. incubating the reaction mixture obtained in ii) to a temperature between 20 and 40° C;
iv. performing a polymerization and crosslinking of the monomers of formula (I) optionally with monomers of formula (II).

2. Method of claim 1, wherein the polyethylene glycol (PEG) polymer contains between 1 and 100% by weight of monomers of formula (I) and between 99 and 0% by weight of monomers of formula (II).

3. Method according to one of claims 1 or 2, wherein the monomers of formula (I) are those wherein $X_1$ and $X_2$ identical or different, are selected from the group consisting of methacrylates or acrylates and n is a number between 2 and 20.

4. Method according to any of claims 1 to 3, wherein the polymerization initiator is a photopolymerization initiator.

5. Method according to claim 4, wherein the photopolymerization initiator is selected from the group comprising Irgacures (2959, 184, 651, 819), 2-hydroxy-4- (2-hydroxyethoxy)-2-methylpropiophenone, benzophenone, 2, 4-dimethylbenzophenone, benzoin, ionic benzophenones selected from the group comprising, 4-trimethylmethylammonium benzophenone chloride, sodium salt of 4-sulfomethylbenzyl, aromatic ketones and aldehydes including benzaldehyde, acetophenone , biacetyl, parachlorobenzophenone, ferulic acid.

6. Method according to any one of claims 1 to 3, wherein the polymerization initiator is a thermal initiator.

7. Method according to claim 6, wherein the thermal initiator is selected from the group comprising peroxides, diazo compounds or persulphates.

8. Method according to any one of claims 1 to 7, wherein in step iii), the incubation temperature is 37 ° C.

9. Method according to any one of claims 1 to 5 and 8, wherein in step iv), the polymerization is carried out under a UV / visible radiation at a wavelength between 190 and 800 nm.

10. Method according to claim 9, wherein in step iv), the polymerization is carried out for 1 to 10 hours.

11. Method according to any of claims 1 to 3 and 6 to 8, wherein the polymerization is carried out at the same time as step (iii).

12. Method according to any of claims 1 to 3 and 6 to 8, wherein the polymerization takes place after step (iv) at a temperature between 25 and 60 °C.

13. Method according to any of claims 1 to 12, wherein after step (iv), the method of the invention further comprises a step of enzymatic crosslinking of the physical fibrin gel.

14. Method according to claim 13, wherein the used enzyme is transglutaminase.

15. Material in form of interpenetrating polymers network (IPN) associating a physical gel of fibrin and a polyethylene glycol (PEG) network obtainable by the method according to any one of claims 1 to 14.

16. Material according to claim 15, wherein the polyethylene glycol (PEG) network contains between 1 and 100% by weight of monomers of formula (I) and between 99 and 0% of monomers of formula (II).

17. Material according to one of claims 15 or 16, wherein the monomers of formula (I) are those wherein $X_1$ and $X_2$, identical or different, are chemical moieties selected from the group comprising methacrylates or acrylates and n is an number between 2 and 20.

18. Material in form of interpenetrating network of polymers (IPN) according to any one of claims 15 to 17, constituted of:

a) from 1 to 50% by dry weight of fibrin;
b) 50 to 99% by dry weight of a polyethylene glycol (PEG) network.

19. Material according to any one of claims 15 to 18, having a glass transition temperature (Tg) of between -100 and + 100 °C.

20. Material according to any one of claims 15 to 19, having a shear modulus (measured in shear mode G ') in the hydrated state at 37 °C between 100 Pa and 10 MPa and a storage modulus (measured in voltage mode E') in the dry state between 0, 01 and 3000 MPa.

21. Use of a material according to any one of claims 15 to 20 or obtainable by the process according to any one of claims 1 to 14, as:

- wound dressing,
- surgical dressing,
- a device to deliver therapeutic agents,
- coating for medical devices selected from the group comprising stents, heart valves, catheters, vascular prosthetic filters,
- support of active molecules selected from the group comprising growth factors, antibiotics, bactericidal, bacteriostatic, or enzymes,
- or for eukaryotic cell culture.

**FIGURES**

550-5   750-5

550-25   750-25

**Figure 1**

Figure 2

Figure 3

Figure 4

Figure 5

Figure 6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **RUSZCZAK, Z.** Effect of collagen matrices on dermal wound healing. *Advanced Drug Delivery Reviews,* 2003, vol. 55, 1595-1611
- **NGUYEN, K. T. ; J. L. WEST.** Photopolymerizable hydrogels for tissue engineering applications. *Biomaterials,* 2002, vol. 23, 4307-4314
- **SCHMEDLEN, R. H. ; J. L. MASTERS et al.** Photocrosslinkable polyvinyl alcohol hydrogels that can be modified with cell adhesion peptides for use in tissue engineering. *Biomaterials,* 2002, vol. 23, 4325-4332
- **DE GENNES, P.-G.** Scaling Concerts in Polymer Physics. Cornell University Press, 1979

- **VERDERIO E. A. M. ; JOHNSON, T. ; GRIFFIN, M.** Tissue transglutaminase in normal and abnormal wound healing. *Amino Acids,* 2004, vol. 26, 387-404
- **W. BENSAÏD ; TRIFFITT J. T. ; BLANCHAT, C. OUDINA ; K. SEDEL, L. ; PETITE, H.** A biodegradable fibrin scaffold for mesenchymal stem cell transplantation. *Biomaterials,* 2003, vol. 24, 2497-2502
- **M.FONTANILLE ; Y.GNANOU.** chimie et physico-chimie des polymères. 2002, 410-413